(19) Europäisches Patentamt
European Patent Office
Office européen des brevets

(11) Veröffentlichungsnummer : **0 117 507**
**B1**

(12) # EUROPÄISCHE PATENTSCHRIFT

(45) Veröffentlichungstag der Patentschrift : **11.11.87**

(51) Int. Cl.$^4$ : **C 07 J 21/00**, C 07 J 1/00, C 07 J 53/00

(21) Anmeldenummer : **84101780.9**

(22) Anmeldetag : **21.02.84**

(54) **Verfahren zur Herstellung von 3-(delta4-3ketosteroid-17alpha-yl)-propion-säurelactonen.**

(30) Priorität : **22.02.83 DE 3306554**

(43) Veröffentlichungstag der Anmeldung : **05.09.84 Patentblatt 84/36**

(45) Bekanntmachung des Hinweises auf die Patenterteilung : **11.11.87 Patentblatt 87/46**

(84) Benannte Vertragsstaaten : **AT BE CH DE FR GB IT LI LU NL SE**

(56) Entgegenhaltungen :
**DE-A- 2 327 448**
**US-A- 3 455 909**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Junghans, Klaus, Dr.**
**Hanstedter Weg 8**
**D-1000 Berlin 41 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Verfahren zur Herstellung von 3-($\Delta^4$-3-Ketosteroid-17α-yl)-propionsäurelactonen der Androstanreihe der allgemeinen Teilformel

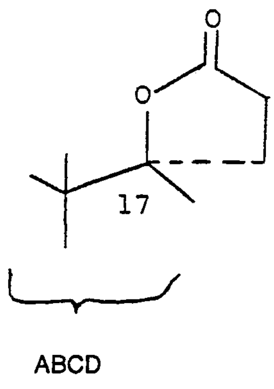

worin ABCD das gegebenenfalls substituierte Cyclopentanophenanthren-Steroidmolekülgerüst der Androstanreihe darstellen soll, aus entsprechenden 17α-(3-Hydroxypropinyl)-17β-hydroxysteroiden der allgemeinen Teilformel

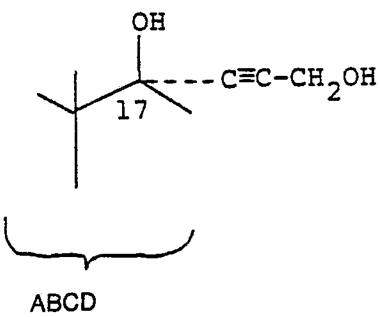

worin ABCD die oben angegebene Bedeutung hat, welches dadurch gekennzeichnet ist, daß man das 17α-(4-Hydroxypropinyl)-17β-hydroxysteroid intermediär zum 17α-(3-Hydroxypropenyl)-17β-hydroxysteroid and Raney-Nickel hydriert, dann mit der zwei bis zwanzigfachen Menge im Vergleich zu der vorher bei der Hydrierung benutzen Menge mit Raney-Nickel behandelt und anschließend mit Chromsäure oxydiert.

Es ist bekannt, daß $\Delta^4$-3-Ketosteroid-17-propiolactone, wie z. B. das 3-(17β-Hydroxy-4-androsten-3-on-17α-yl)-propionsäurelacton, durch homogene Hydrierung der entsprechenden 17α-Hydroxypropinyl-$\Delta^4$-3-ketosteroide zu den entsprechenden 17α-Hydroxypropyltestosteronen und nachfolgende Jones-Oxydation in einer zweistufigen Reaktion hergestellt werden können (DE AS 2,327,448).

Die Hydrierung mit homogenen Katalysatoren, wie mit Tris-triphenylphosphin-rhodiumchlorid, hat neben dem hohen Katalysatorpreis den Nachteil, daß das aus toxikologischer Sicht nicht unbedenkliche Rhodium nur sehr aufwendig aus den Folgestufen entfernt werden kann.

Es ist ferner bekannt, daß man 17α-Hydroxypropinyl-$\Delta^{1,4}$-3-ketosteroide selektiv mittels Wasserstoff in Gegenwart von Palladium-Calziumcarbonat-Katalysatoren zu den 17α-Hydroxypropenyl-$\Delta^{1,4}$-3-ketosteroiden hydrieren kann. Diese Verbindungen werden isoliert und in einem Reaktionsschritt mittels Chromsäure-Schwefelsäure oxidierend cycliert. (US-Patentschrift 3,455,909).

Die Aufgabe der vorliegenden Erfindung bestand darin, ein vereinfachtes Verfahren zur Herstellung von 4-3-Ketosteroidpropiolacton der Androstanreihe unter Verwendung eines Hydrierkatalysators zu finden.

Überraschenderweise wurde nun gefunden, daß das intermediär gebildete Propenol 17α-(3-Hydroxypropenyl)-17β-hydroxysteroid in Gegenwart von Raney-Nickel in das entsprechende Lactol umgelagert wird, das dann ohne Abtrennung weiter zum Lacton oxidiert wird.

Eine derartige Allylumlagerung an Raney-Nickel ist bisher noch nicht beschrieben worden. Eine so glatte Reaktion war bei so komplizierten Molekülen wie den vorliegenden Steroiden nicht zu erwarten gewesen. Mit Raney-Nickel wäre eher eine Reaktion der $\Delta^4$- oder $\Delta^{20}$-Doppelbindung zu erwarten gewesen.

Das erfindungsgemäße Verfahren läuft schematisch nach folgenden Schema ab :

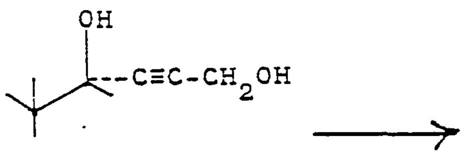

Hydroxypropinol

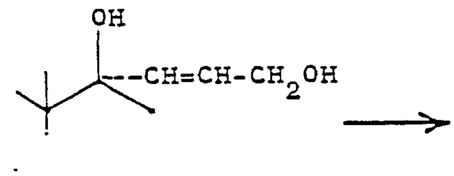

Hydroxypropenol

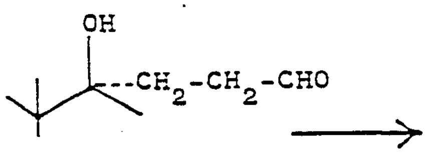

Hydroxy-propylaldehyd

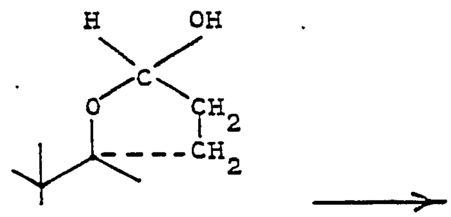

Propiolactol

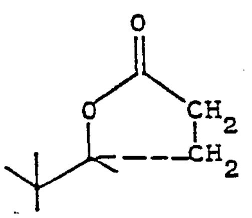

Propiolacton

Das erfindungsgemäße Verfahren wird zweckmäßigerweise so durchgeführt, daß man das 17α-(3-Hydroxypropinyl)-17β-hydroxysteroid in einem geeigneten Lösungsmittel löst und an Raney-Nickel bis zur Aufnahme eines Äquivalents Wasserstoff hydriert.

Zur Hydrierung wird eine 0,05-0,2 fache Katalysatormenge, bezogen auf eingesetztes Steroid, benutzt. Das verwendete Raney-Nickel ist handelsüblicher Qualität wie z. B. die Typen B 113 Z und B 115 Z der Firma Degussa, Frankfurt/Main. Die Hydrierung kann bei Normaldruck ausgeführt werden, jedoch verläuft sie unter leicht erhöhtem Druck im Bereich von 1-5 bar entsprechend schneller. Die Reaktionstemperatur beträgt etwa 0 bis 50 °C.

Anschließend wird das Reaktionsgemisch mit einer größeren Menge des gleichen Raney-Nickels, das zuvor auch zur Hydrierung verwendet wurde, versetzt. Die Menge beträgt etwa das 2-20 fache der vorher zugesetzten Menge.

Die Reaktionsdauer ist abhängig von der Reaktionszeit, sie beträgt mindestens eine Stunde.

Nach der Katalysatorzugabe wird noch einige Zeit gerührt, wobei die Zeit davon abhängt, bei welcher Reaktionstemperatur gerührt wird. Bei Temperaturen um 0 °C beträgt die Reaktionszeit etwa 8 Stunden, bei der Temperatur des siedenden Reaktionsgemisches etwa eine 1/4 Stunde. Zweckmäßigerweise arbeitet man aber bei Raumtemperatur und benötigt dann eine Reaktionszeit von 1-3 Stunden.

Anschließend wird das Reaktionsgemisch, nachdem der Katalysator abfiltriert ist, mit Chromsäure in einem geeigneten Reaktionsmedium wie Eisessig, Schwefelsäure/Aceton oder Schwefelsäure/Wasser behandelt. Die Reaktion wird zweckmäßigerweise so ausgeführt, daß man die Chromsäurelösung langsam und unter Kühlung zu dem Reaktionsgemisch gibt.

Die erforderliche Menge Chromsäure beträgt 1 bis 1,5 Äquivalent bezogen auf das Steroid. Die Reaktionstemperatur liegt bei 0 bis 20 °C. Die Reaktionszeit bei 10 Minuten bis einer Stunde.

Als Lösungsmittel kommen für die Hydrierung und die anschließende Umlagerungsreaktion an sich nur aprotische Lösungsmittel in Frage, da in einem protischen Lösungsmittel, wie in einem Alkohol, Nebenreaktionen wie die Reduktion der $\Delta^4$-Doppelbindung auftreten würden.

Als aprotische Lösungsmittel seien beispielsweise genannt Aceton, Methylisobutylketon, Benzol, Toluol, Dioxan, Tetrahydrofuran, Dimethylformamid und deren Gemische untereinander.

Die Aufarbeitung des Reaktionsproduktes erfolgt nach den üblicherweise in der Chemie angewandten Arbeitsmethoden, wie zum Beispiel durch Extraktion oder durch Einengen im Vakuum. Das erhaltene Rohprodukt wird in üblicher Weise, wie zum Beispiel durch Umkristallisation oder Chromatographie, gereinigt.

Das als Ausgangsmaterial verwendete 17α-(3-Hydroxypropinyl)-17β-hydroxysteroid kann an sich an den Ringen A, B, C und D in beliebiger Weise substituiert sein, solange die Substituenten gegenüber den Reaktanten inert sind. So können sich beispielsweise Methylgruppen in 1-, 6- und 16-Stellung, Methylengruppen in 1.2-, 6.7- und 15.16-Stellung oder Oxogruppen beispielsweise in der 3-Stellung besitzen. Das Ausgangssteroid kann gesättigt oder beispielsweise in der 4(5), 5(6), 6(7), 9(11) oder 15(16)-Stellung ungesättigt sein. 3-Hydroxy-$\Delta^5$-steroide, 3,5-Dihydroxysteroide und Ketale oder Enolether von 3-Keto-$\Delta^4$-steroiden werden während der Reaktion in 3-Keto-$\Delta^4$-steroide überführt.

Das erfindungsgemäße Verfahren hat insbesondere den Vorteil, daß es technisch einfach durchzuführen ist.

Die nach den erfindungsgemäßen Verfahren herstellbaren Verbindungen, insbesondere die der allgemeinen Formel I gemäß Patentanspruch 1 sind Zwischenprodukte zur Herstellung von Aldosteron-Antagonisten. So ist das 3-(17β-Hydroxy-4-androsten-3-on-17α-yl)-propionsäurelacton selbst biologisch aktiv und wird durch Dehydrierung in 6-Stellung gemäß J. Org. Chem. 24, (1959) 1109 und anschließende Umsetzung mit Thioessigsäure gemäß z. B. DT AS 1.121.610 in den bekannten Aldosteron-Antagonisten 3-(7α-Acetyl-thio-17β-hydroxy-3-oxo-4-androsten-17α-yl)-proppionsäurelacton (Spironolacton) überführt.

In analoger Weise erhält man aus dem 3-(17β-Hydroxy-6β.7β ; 15β.16β-bis-methylen-4-androsten-3-on-17α-yl)-propio-lacton das bekannte 6β.7β ; 15β.16β-Dimethylen-3-oxo-4-androsten-[17-(β-1')-spiro-5'] perhydrofuran-2'-on (DE OS 2 652 761). Die übrigen Verfahrensprodukte können beispielsweise nach den Verfahren der US-Patentschriften 3,013,012, 3,966,714 und 4,129,564 sowie nach den in den europäischen Patentanmeldungen 83730074.8 und 83730098.7 beschriebenen Verfahren in pharmakologisch wirksame Steroide überführt werden.

Die nachfolgenden Beispiele sollen das erfindungsgemäße Verfahren erläutern.

Beispiel 1

3,0 g 17α-(3-Hydroxypropinyl)-17β-hydroxy-4-androsten-3-on werden in 50 ml Tetrahydrofuran mit 0,3 g Raney-Nickel (Firma Degussa, Typ B 113 Z) 1,5 Stunden hydriert. Zu der Reaktionslösung werden weitere 3 g Raney-Nickel gegeben und 3 Stunden bei Raumtemperatur gerührt, filtriert und die Lösung mit 2,5 ml Jones-Lösung (hergestellt aus 4,6 ml konzentrierter Schwefelsäure und 5,4 mg Chromtrioxid mit Wasser auf 20 ml aufgefüllt) bei 0 °C versetzt. Nach 5 Minuten wird die überschüssige Chromsäure mit Methanol versetzt, 100 ml Eiswasser zugegeben und mit Methylenchlorid extrahiert. Der Extrakt wird mit Wasser gewaschen, über Magnesiumsulfat getrocknet und eingedampft. Der ölige Rückstand wird mit 5 ml Diisopropylether kurz erhitzt, wobei Kristallisation eintritt, und im Vakuum zur Trockne eingedampft. Man erhält 2,4 g 3-(17β-Hydroxy-4-androsten-3-on-17α-yl)-propionsäurelacton vom Schmelzpunkt 153-155 °C.

Beispiel 2

3,0 g 17α-(3-Hydroxypropinyl)-17β-hydroxy-4-androsten-3-on werden in 50 ml Aceton mit 0,3 g Raney-Nickel (Firma Degussa, Typ B 115 Z) hydriert. Nach Zugabe von weiteren 2 g Raney-Nickel wird 15 Minuten am Rückfluß erhitzt, auf Raumtemperatur abgekühlt, filtriert und bei 0 °C mit 2,5 ml Jones-Lösung versetzt. Die Aufarbeitung des Reaktionsgemisches erfolgt wie im Beispiel 1 beschrieben. Man erhält 2,3 g 3-(17β-Hydroxy-4-androsten-3-on-17α-yl)-propionsäurelacton vom Schmelzpunkt 152-156 °C.

Beispiel 3

5,0 g 17α-(3-Hydroxypropionyl)-17β-hydroxy-6.7 ; 15.16-bis-methylen-4-androsten-3-on werden in 50 ml Toluol mit 0,3 g Raney-Nickel hydriert, (Degussa Typ B 115 Z). Dann setzt man noch 2 g Raney-Nickel zu und erhitzt die Reaktionsmischung 20 Minuten lang. Dann wird die Mischung einge-dampft, der Rückstand in Aceton aufgenommen und mit 2,5 ml Jones Reagens bei 0 °C oxidiert. Nach Aufarbeitung wie in Beispiel 1 beschrieben erhält man 4,8 g 3-(17β-Hydroxy-6,7 ; 15.16-bis-methylen-4-androsten-3-on-17α-yl)-propionsäurelacton vom Schmelzpunkt 193-195 °C.

Herstellung des Ausgangsmaterials

1,0 g 3β.5β-Dihydroxy-6.7β ; 15.16β-dimethylen-androstan-17-on (Angew. Chem. 94,718 (1982)) werden in 10 ml Dimethylformamid mit 2 g Pyridiniumdichromat 7 Stunden auf 70 °C erwärmt, in 100 ml Essigsäureethylester eingerührt, über Natriumsulfat filtriert, das Filtrat mit verdünnter Schwefelsäure, Wasser, Natriumbicarbonatlösung gewaschen, getrocknet und eingedampft. Der Rückstand (0,76 g) ergibt nach Kristallisieren aus Aceton 0,3 g farblose Kristalle vom Schmelzpunkt 229 °C.

$[\alpha]_D = -225,7°$

2,17 g 6.7 ; 15.16-Dimethylen-androst-4-en-3.17-dion werden in 20 ml Tetrahydrofuran unter Stickstoff

mit 8,84 g Kaliumethylat versetzt und unter Rühren 2 ml Propargylalkohol in 2 ml Tetrahydrofuran zugetropft. Nach 30 Minuten bei Raumtemperatur wird mit Essigsäure neutralisiert, eingeengt, Wasser zugegeben und mit Methylenchlorid ausgeschüttelt, dieses über Magnesiumsulfat getrocknet und eingedampft. Der Rückstand (2,68 g) ergibt nach Chromtographie an Silicagel 440 mg farblose Kristalle vom Schmelzpunkt 165 °C.

$[\alpha]_D = -311{,}4°$ (Chloroform).

Beispiel 4

1,0 g 17α-(3-Hydroxypropinyl)-3β.5β.17β-trihydroxy-6,7 ; 15.16-bis-methylen-androstan (Angew. Chem. 94, 718 (1982)) werden in 25 ml Tetrahydrofuran mit 0,2 g Raney-Nickel hydriert. Nach Zugabe von 2,5 g Raney-Nickel wird 30 Minuten gerührt, filtriert und bei 0 °C mit überschüssiger Jones-Lösung versetzt. Die Aufarbeitung erfolgt wie in Beispiel 1 beschrieben und ergibt 0,5 g 3-(17β-Hydroxy-6.7 ; 15.16-bis-methylen-4-androsten-3-on-17α-yl)-propionsäurelacton vom Schmelzpunkt 190-194 °C.

**Patentansprüche**

1. Verfahren zur Herstellung von 3-($\Delta^4$-3-Ketosteroid-17α-yl)-propionsäurelactonen der Androstanreihe der allgemeinen Teilformel

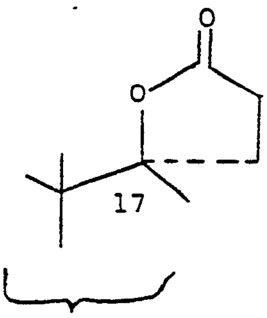

ABCD

worin ABCD das gegebenenfalls substituierte Cyclopentanophenanthren-Steroidmolekülgerüst der Androstanreihe darstellen soll, aus entsprechenden 17α-(3-Hydroxy-propinyl)-17β-hydroxy-steroiden der allgemeinen Teilformel

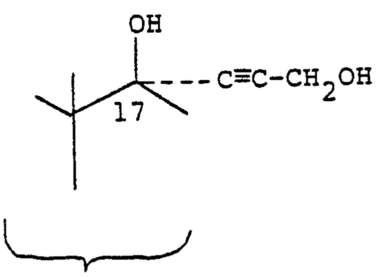

ABCD

worin ABCD die oben angegebene Bedeutung hat, dadurch gekennzeichnet, daß man das 17α-(4-Hydroxypropinyl)-17β-hydroxysteroid intermediär zum 17α-(3-Hydroxypropenyl)-17β-hydroxysteroid an Raney-Nickel hydriert, dann mit der zwei bis zwanzigfachen Menge im Vergleich zu der vorher bei der Hydrierung benutzten Menge mit Raney-Nickel behandelt und anschließend mit Chromsäure oxydiert.

2. Verfahren zur Herstellung von 3-($\Delta^4$-3-Ketosteroid-17α-yl)-propionsäurelactonen der allgemeinen Formel I

(Siehe Formel I Seite 6 f.)

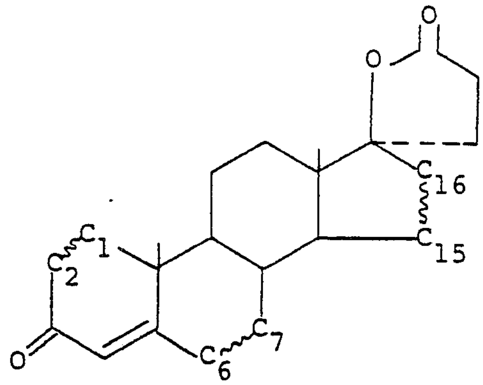

(I)

worin $-C_1 \sim C_2-$, $-C_6 \sim C_7-$ und $-C_{15} \sim C_{16}-$ gleich oder verschieden sind und die Gruppierungen $-CH_2-CH_2-$, $-CH=CH-$ oder

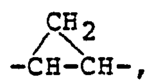

bedeuten, gemäß Patentanspruch 1, dadurch gekennzeichnet, daß man ein 17α-(3-Hydroxypropinyl)-17β-hydroxysteroid der allgemeinen Formel II

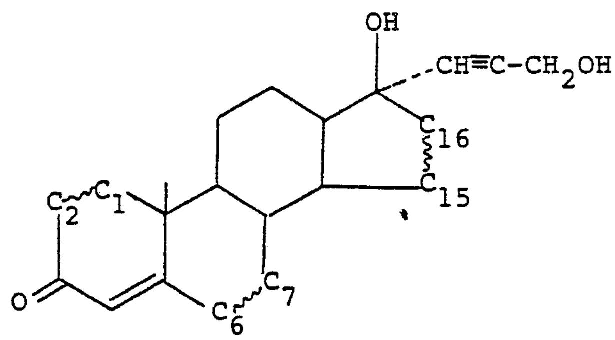

(II)

worin $-C_2 \sim C_1-$, $-C_6 \sim C_7-$ und $-C_{15} \sim C_{16}-$ die obengenannte Bedeutung besitzen, als Ausgangsmaterial verwendet.

3. Verfahren zur Herstellung von 3-($\Delta^4$-3-Ketosteroid-17α-yl)-propionsäurelactonen gemäß Patentanspruch 1 und 2, dadurch gekennzeichnet, daß man die Reaktion in einem aprotischen Lösungsmittel durchführt.

## Claims

1. Process for the preparation of 3-($\Delta^4$-3-ketosteroid-17α-yl)-propionic acid lactones of the androstane series of the partial general formula

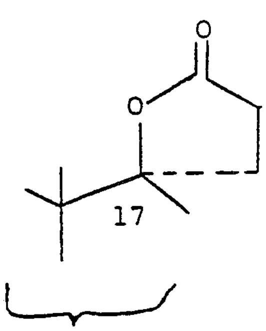

ABCD

in which ABCD is to represent the optionally substituted cyclopentanophenanthrene steroid molecular structure of the androstane series, from corresponding 17α-(3-hydroxypropynyl)-17β-hydroxysteroids of the partial general formula

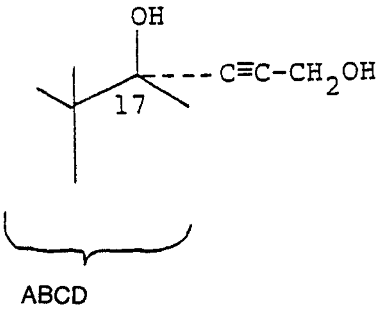

in which ABCD has the meaning given above, characterised in that the 17α-(4-hydroxypropynyl)-17β-hydroxysteroid is hydrogenated intermediately on Raney nickel to form the 17α-(3-hydroxypropenyl)-17β-hydroxysteroid, is then treated with from two to twenty times the amount of Raney nickel as that used previously in the hydrogenation, and is subsequently oxidised with chromic acid.

2. Process for the preparation of 3-($\Delta^4$-3-ketosteroid-17α-yl)-propionic acid lactones of the general formula I

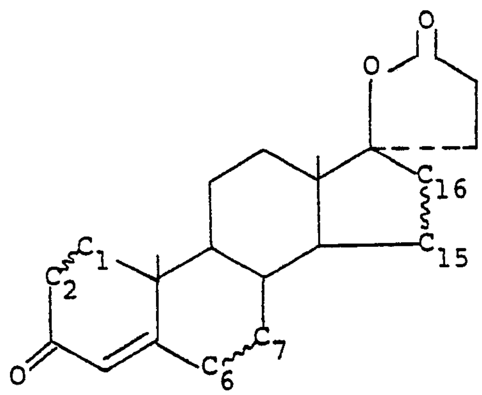

(I)

in which $-C_1$ ~~~ $C_2-$, $-C_6$ ~~~ $C_7-$ and $-C_{15}$ ~~~ $C_{16}-$ are the same or different and represent the groupings $-CH_2-CH_2-$, $-CH{=}CH-$ or

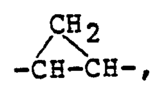

according to patent claim 1, characterised in that a 17α-(3-hydroxypropynyl)-17β-hydroxysteroid of the general formula II

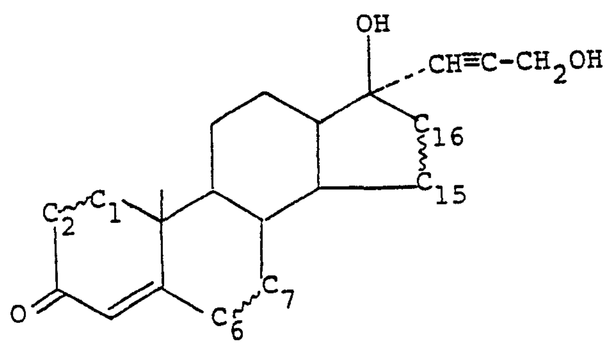

(II)

in which $-C_2$ ~~~ $C_1-$, $-C_6$ ~~~ $C_7-$ and $-C_{15}$ ~~~ $C_{16}-$ have the meanings given above, is used as starting material.

3. Process for the preparation of 3-($\Delta^4$-3-ketosteroid-17α-yl)-propionic acid lactones according to patent claims 1 and 2, characterised in that the reaction is carried out in an aprotic solvent.

**Revendications**

1. Procédé de préparation d'(oxo-3$\Delta^4$-stéroïdyl-17α)-3 propionolactones de la série de l'androstane qui répondent à la formule générale partielle suivante :

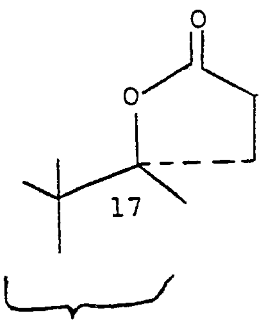

ABCD

dans laquelle ABCD représente le système cyclique cyclopentanophénanthrénique, éventuellement substitué, de la molécule d'un stéroïde appartenant à la série de l'androstane, à partir des (hydroxy-3 propynyl)-17α hydroxy-17β stéroïdes correspondants de formule générale partielle suivante :

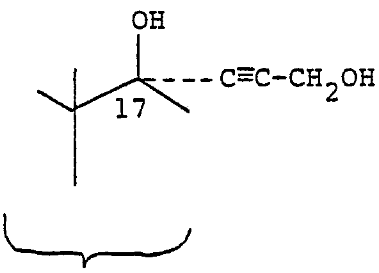

ABCD

dans laquelle ABCD a la signification indiquée ci-dessus, procédé caractérisé en ce qu'on hydrogène intermédiairement, en présence de nickel Raney, l'(hydroxy-4 propynyl)-17α hydroxy-17β stéroïde pour le convertir en (hydroxy-3 propényl)-17α hydroxy-17β stéroïde, puis on traite ce dernier avec une quantité de nickel de Raney représentant de 2 à 20 fois celle qui a été utilisée auparavant lors de l'hydrogénation, et on oxyde ensuite par de l'acide chromique.

2. Procédé de préparation d'(oxo-3 $\Delta^4$ stéroïdyl-17α)-3 propionolactones répondant à la formule générale I :

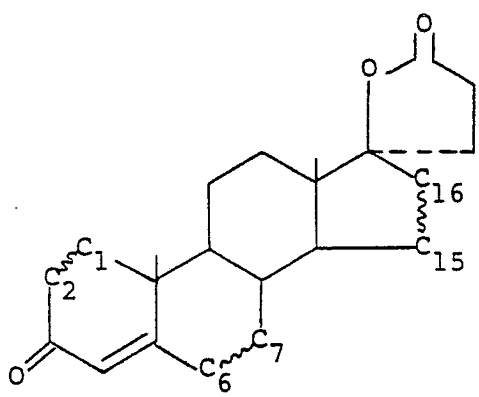

(I)

dans laquelle $-C_1 \sim\sim C_2-$, $-C_6 \sim\sim C_7-$ et $-C_{15} \sim\sim C_{16}-$, qui peuvent être identiques ou différents, représentent chacun, indépendamment les uns des autres, l'un des groupements ; $-CH_2-CH_2-$, $-CH{=}CH-$ et

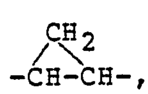

selon la revendication 1, procédé caractérisé en ce qu'on utilise, comme corps de départ, un (hydroxy-3 propynyl)-17α hydroxy-17β stéroïde répondant à la formule générale II :

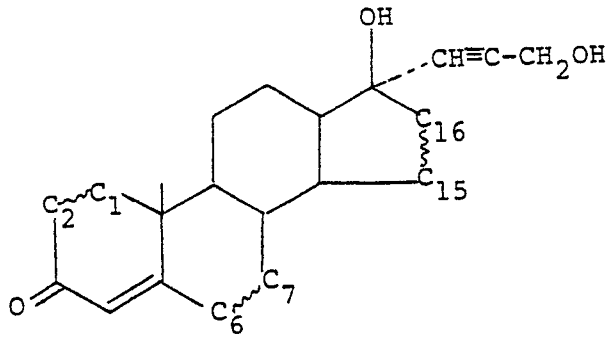

(II)

dans laquelle $-C_2 \sim\sim C_1-$, $-C_6 \sim\sim C_7-$ et $-C_{15} \sim\sim C_{16}-$ ont les significations indiquées ci-dessus.

3. Procédé de préparation d'(oxo-3$\Delta^4$ stéroïdyl-17α)-3 propionolactones selon l'une des revendications 1 et 2, procédé caractérisé en ce qu'on effectue la réaction dans un solvant aprotique.